# EUROPÄISCHE PATENTSCHRIFT

(11) **EP 0 839 786 B1**
(45) Veröffentlichungstag und Bekanntmachung des Hinweises auf die Patenterteilung: **28.08.2002**
(21) Anmeldenummer: 97113105.7
(22) Anmeldetag: 30.07.1997
(51) Int. Cl.: C07C 29/151, C07C 31/04, C01B 3/38

(54) **Verfahren zum Erzeugen von Methanol aus Erdgas**
Method for the preparation of methanol from natural gas
Procédé pour préparation de méthanol du gaz naturel

(30) Priorität: 24.10.1996 DE 19644216
(43) Veröffentlichungstag der Anmeldung: 06.05.1998
(73) Patentinhaber: MG Technologies AG, 60325 Frankfurt am Main (DE)
(72) Erfinder: Boll, Walter, Dr., 60388 Frankfurt am Main (DE); Göhna, Hermann, 65812 Bad Soden (DE)
(74) Vertreter: Revesz, Veronika

(56) Entgegenhaltungen:
- EP-A- 0 123 534
- EP-A- 0 233 076
- GB-A- 2 142 331
- US-A- 5 310 506
- HOLM-LARSEN H ET AL: "AUTOTHERMAL REFORMING TURNS METHANOL PLANT OFF-GAS INTO A LOW COST FEEDSTOCK" NITROGEN, Nr. 222, 1. Juli 1996 (1996-07-01), Seiten 37-40, XP000598595 ISSN: 0029-0777
- "LOW COST ROUTES TO HIGHER METHANOL CAPACITY" NITROGEN, Nr. 224, 1. November 1996 (1996-11-01), Seiten 31/32, 34-36, 38, XP000639347 ISSN: 0029-0777

## Beschreibung

Die Erfindung betrifft ein Verfahren zum Erzeugen von Methanol aus Erdgas, wobei man das Erdgas mit Wasserdampf und molekularem Sauerstoff katalytisch umsetzt und ein rohes Synthesegas aus der katalytischen Umsetzung abzieht, welches H₂, CO und CO₂ enthält, wobei man dem rohen Synthesegas ein H₂-reiches Gas und Kreislaufgas zumischt und ein Synthesegasgemisch erzeugt, welches man durch mindestens einen Methanolsynthesereaktor leitet, der einen Kupferkatalysator enthält und bei einem Druck im Bereich von 10 bis 150 bar und Temperaturen im Bereich von 180 bis 350°C betrieben wird, wobei man aus dem Methanolsynthesereaktor ein Methanoldampf enthaltendes Gasgemisch abzieht, welches man soweit kühlt, daß Methanol auskondensiert, wobei ein gekühltes Restgas getrennt vom Methanol abgezogen und in das Kreislaufgas und in einen zweiten Teilstrom (Purgegas) geteilt wird, wobei man aus dem Purgegas, welches einen H₂-Gehalt von mindestens 30 Vol.% (trocken gerechnet) aufweist, in einer Trenneinrichtung ein H₂-reiches Gas abtrennt und es dem rohen Synthesegas zumischt.

Ein solches Verfahren ist aus EP-B 0 233 076 bekannt. Hierbei wird das Erdgas oder ein Teilstrom des Erdgases zunächst durch eine Dampfreformierung (Steam Reforming) geleitet, und man gibt dann das so erzeugte Spaltgas zusammen mit restlichem Erdgas einem Katalysator enthaltenden, autotherm betriebenen Reaktor auf.

Die Dampfreformierung erfolgt, wie üblich, im Röhrenofen, wobei ein Nickelkatalysator in zahlreichen Röhren angeordnet ist, die sich in einer Brennkammer befinden und durch heißes Verbrennungsgas von außen beheizt werden. Die Dampfreformierung des bekannten Verfahrens dient dazu, den Wasserstoffgehalt im nachfolgenden autothermen Reaktor zu erhöhen und auch für einen erhöhten Wasserstoffgehalt im erzeugten rohen Synthesegas zu sorgen.

Die EP-A-0533231 beschreibt eine autotherm betriebene Methanolerzeugung, wobei zur Anreicherung des Synthesegases Wasserstoff aus einer anderen Anlage zur Verfügung gestellt wird. Reicht diese H₂-Menge zur Einstellung des notwendigen Verhältnisses von H₂ zu Kohlenoxiden noch nicht aus, wird zusätzlich aus der Methanolsynthese ein wasserstoffreiches Restgas abgeführt und in den Reformierreaktor eingeleitet, oder ein wasserstoffreiches Gas einer Trennanlage zugeführt und danach dem Synthesegas beigemischt.

Die Veröffentlichung von Holm-Larsen et al "Autothermal Reforming Turns Methanol Plant Off-Gas into a low cost Feedstock" beschreibt die Erweiterung einer bestehenden Methanolanlage durch eine "add-on unit". Das H₂ -Defizit der nachträglich installierten Parallelanlage wird aus den Restgasen der ursprünglichen Anlage und der hinzugefügten Anlage gedeckt. Ein wesentlicher Teil des in der hinzugefügten Anlage benötigten H₂ wird auch aus einer Fremdanlage bezogen.

Der Erfindung liegt die Aufgabe zugrunde, das bekannte Verfahren so abzuwandeln, daß das Verfahren mit niedrigen Investitionskosten realisiert werden kann und gleichzeitig ein günstiger Verbrauch an Prozeßmitteln erreicht wird. Dabei soll auch auf die Dampfreformierung verzichtet werden können. Erfindungsgemäß geschieht dies beim eingangs genannten Verfahren dadurch, daß
(a) man die katalytische Umsetzung des Erdgases mit dem Wasserdampf und dem Sauerstoff nur im autotherm betriebenen Reaktor durchführt, der mindestens einen, mit dem Erdgas und dem Sauerstoff gespeisten Brenner und unter dem Brenner eine Schüttung aus körnigem Katalysator aufweist;
(b) man das rohe Synthesegas mit einer Temperatur im Bereich von 800 bis 1200°C aus dem autotherm betriebenen Reaktor abzieht, kühlt, Wasserdampf auskondensiert und gebildetes Kondensat abtrennt;
(c) das rohe, gekühlte Synthesegas vor der Zumischung des H₂-reichen Gases eine Stöchiometriezahl von 1,3 bis 1,9 hat, wobei sich S aus den molaren Konzentrationen von H₂, CO und CO₂ gemäß
   S = (H₂ - CO₂) : (CO + CO₂) errechnet;
(d)
   - das Purgegas: zu 30 bis 80 Vol.% aus H₂,
   zu 7 bis 35 Vo.% und vorzugsweise
   zu mindestens 10 Vol.% aus CO₂ und
   zu 3 bis 25 Vol.% aus CO besteht,
(e) die stündliche Menge an Purgegas 8 bis 25 % und vorzugsweise 10 bis 15 % (trocken gerechnet) der stündlichen Menge an gekühltem, rohem Synthesegas beträgt;
(f) die im Purgegas zur Trenneinrichtung geführte stündliche Menge an CO₂ 10 bis 50 % und vorzugsweise 15 bis 35 % der stündlichen CO₂-Menge im rohen Synthesegas beträgt, und
(g) die im Purgegas zur Trenneinrichtung geführte stündliche Menge an CO 2 bis 10 % der stündlichen CO-Menge im rohen Synthesegas beträgt,
(h) das aus der Trenneinrichtung abgezogene, dem rohen Synthesegas zugemischte H₂-reiche Gas einen H₂-Gehalt von mindestens 80 Vol.% hat, wobei durch die H₂-Zumischung im Synthesegas eine Stöchiometriezahl S von 1,95 bis 2,2 erreicht wird.

Beim erfindungsgemäßen Verfahren wird dafür gesorgt, daß das Purgegas einen relativ großen Gasstrom darstellt. Dies wird dadurch erreicht, daß man den Methanolsynthesereaktor so betreibt, daß neben dem Methanoldampf ständig ein großer Restgasstrom abgezogen werden kann. Man verzichtet dabei bewußt darauf, im Synthesereaktor einen optimal hohen Umsatz an H₂, CO und CO₂ zu erreichen. Insbesondere wird der Umsatz an CO₂ verringert, wodurch auch der H₂-Umsatz erheblich verringert wird. Beim Verfahren der Erfindung sorgt man dafür, daß die Purgegas-Menge mindestens die Menge an Wasserstoff enthält, die zur notwendigen Anhebung der Stöchiometriezahl des rohen Synthesegases nötig ist. Im Methanolsynthesereaktor wird die notwendige Katalysatormenge kleiner, so daß der Methanolsynthesereaktor kleiner ausgeführt werden kann. Ebenso ist es möglich, den Reaktor mit relativ niedrigem Druck oder mit niedrigem Kreislaufverhältnis zu betreiben. Zu diesen, die Kosten der Anlage erheblich verringernden Vorteilen kommt hinzu, daß man auch ohne CO-Konvertierung und ohne CO₂-Entfernung im rohen Synthesegas arbeitet. Da man erfindungsgemäß auf die Dampfreformierung verzichtet, kann man auch im autotherm betriebenen Reaktor grundsätzlich bei höherem Druck arbeiten. Da gleichzeitig der Druck im Methanolsynthesereaktor relativ niedrig sein kann, ergibt sich so die Möglichkeit einer weitgehenden Angleichung der Drücke in der Gaserzeugung und der Synthese.

Der autotherm betriebene Reaktor, der ohne indirekte Beheizung des Katalysators arbeitet, ist bekannt und z.B. in Ullmann's Encyclopedia of Industrial Chemistry, 5. Auflage, Band A 12, Seiten 202 bis 204 beschrieben. Das Erdgas wird dem Reaktor vorzugsweise vorgewärmt zugeführt. Üblicherweise führt man dem Brenner des Reaktors technisch reinen Sauerstoff zu, um den Gehalt an Inertgas im rohen Synthesegas möglichst niedrig zu halten. Die Zufuhr an Wasserdampf liegt üblicherweise im Bereich von 0,5 bis 3,0 Mol bezogen auf den molaren Kohlenstoffgehalt des Erdgases. Der autotherm betriebene Reaktor kann einteilig oder mehrstufig ausgebildet sein. Anstelle von Erdgas kann der autotherm betriebene Reaktor auch mit anderen Einsatzstoffen, z.B. mit Flüssiggas oder Raffineriegas beschickt werden.

Für den Methanolsynthesereaktor kommen an sich bekannte Konstruktionen infrage, insbesondere der wassergekühlte Röhrenreaktor oder der adiabatisch betriebene Festbettreaktor.

Die Trenneinrichtung zum Erzeugen des H₂-reichen Gases wird ebenfalls in an sich bekannter Weise ausgestaltet, z.B. als Druckwechseladsorptionsanlage oder Tieftemperatur-Gaszerlegung.

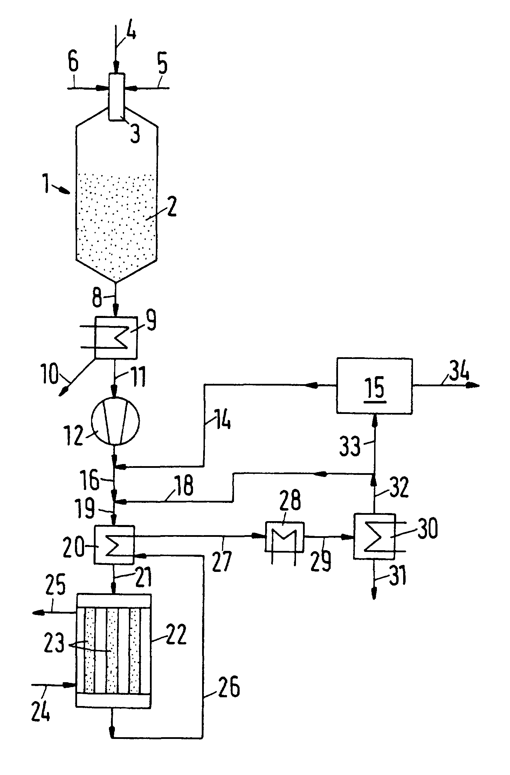

Ausgestaltungsmöglichkeiten des Verfahrens werden mit Hilfe der Zeichnung erläutert. Die Zeichnung zeigt ein Fließschema des Verfahrens.

Der autotherm betriebene Reaktor (1) weist eine Schüttung (2) aus körnigem Nickelkatalysator auf. Über der Schüttung befindet sich ein Brenner (3), dem man durch die Leitung (4) entschwefeltes Erdgas, durch die Leitung (5) O₂-reiches Gas und durch die Leitung (6) Wasserdampf zuführt. Als O₂-reiches Gas wird üblicherweise technisch reiner Sauerstoff verwendet. Die für die Umsetzung im Reaktor (1) nötige Wärme wird allein nur durch partielle Oxydation aufgebracht. Die Temperaturen am Auslaß (8) des Reaktors (1) liegen im Bereich von 800 bis 1200°C und üblicherweise bei mindestens 900°C.

Heißes, rohes Synthesegas verläßt den Reaktor (1) und strömt durch eine Kühlung (9), die mehrstufig ausgebildet sein kann, was in der Zeichnung nicht im einzelnen dargestellt ist. Bei der Kühlung fällt ein wäßriges Kondensat an, welches in der Leitung (10) abgezogen wird. Das gekühlte, rohe Synthesegas, das durch die Leitung (11) strömt, weist üblicherweise Temperaturen im Bereich von 20 bis 80°C auf. Dieses Gas wird von einem Kompressor (12) verdichtet. Durch die Leitung (14) wird H₂-reiches Gas herangeführt, das aus einer Trenneinrichtung (15) kommt. Der H₂-Gehalt im Gas der Leitung (14) beträgt mindestens 80 Vol.% und vorzugsweise mindestens 90 Vol.%.

Die Stöchiometriezahl S des Gases in der Leitung (11) liegt im Bereich von 1,3 bis 1,9 und zumeist bei höchstens 1,8. Durch Zumischen des H₂reichen Gases der Leitung (14) erhöht sich die Stöchiometriezahl im Synthesegas der Leitung (16) auf 1,95 bis 2,2, wie dies für die Methanolsynthese nötig ist. S errechnet sich in bekannter Weise aus den molaren Konzentrationen von
H₂, CO und CO₂ gemäß S = (H₂ - CO₂) : (CO + CO₂).

Dem Gas in der Leitung (16) wird durch die Leitung (18) Kreislaufgas zugemischt und das so gebildete Synthesegasgemisch wird in der Leitung (19) zunächst zu einem Wärmeaustauscher (20) geführt, bevor es durch die Leitung (21) in den Methanolsynthesereaktor (22) eintritt.

In der Zeichnung ist der Reaktor (22) als an sich bekannter Röhrenreaktor dargestellt, bei dem der Kupferkatalysator in zahlreichen Röhren (23) angeordnet ist. Zum indirekten Kühlen des Innenbereichs der Röhren (23) führt man dem Reaktor durch die Leitung (24) Kühlwasser zu und zieht erhitztes und teilweise verdampftes Kühlmedium durch die Leitung (25) ab. In den Röhren (23) liegen die Temperaturen im Bereich von 180 bis 350°C und zumeist im Bereich von 200 bis 300°C. Der Druck in den Röhren beträgt 10 bis 150 bar und liegt zumeist im Bereich von 20 bis 100 bar.

Aus dem Reaktor (22) zieht man durch die Leitung (26) ein Methanoldampf enthaltendes Gasgemisch ab, welches man zunächst im Wärmeaustauscher (20) kühlt und dann durch die Leitung (27) zu einem indirekten Kühler (28) führt. Durch die Leitung (29) gelangt das Gemisch zum Kondensator (30), aus welchem man durch die Leitung (31) ein Methanol und Wasser enthaltendes Kondensat abzieht. Die weitere destillative Behandlung dieses Kondensats, die an sich bekannt ist, braucht hier nicht näher erläutert zu werden.

Aus dem Kondensator (30) zieht man durch die Leitung (32) gekühltes Restgas ab und teilt es in zwei Gasströme. Der erste Strom wird als Kreislaufgas durch die Leitung (18) zurückgeführt. Der zweite Strom, der hier Purgegas genannt wird, wird in der Leitung (33) der Trenneinrichtung (15) aufgegeben, die z.B. nach dem Prinzip der Druckwechseladsorption arbeitet. In der Trenneinrichtung (15) fällt neben dem H₂-reichen Gas der Leitung (14) ein Methan, CO und CO₂ enthaltendes Abgas an, das in der Leitung (34) abzieht und als Brenngas verwendbar ist oder einer anderweitigen Nutzung zugeführt werden kann.

Um die Gase der Leitungen (14) und (18) dem Synthesegas zumischen zu können, wird ein Verdichter nötig sein, der jedoch zur Vereinfachung in der Zeichnung weggelassen wurde. Den Reaktor (1) kann man bei einem Druck oberhalb von 40 bar betreiben, wobei der Kompressor (12) entfallen kann.

### Beispiele:

Die folgenden Beispiele, die teilweise berechnet sind, haben die Erzeugung von 1000 Tagestonnen Methanol zum Ziel. Der Nickelkatalysator im Reaktor (1) besteht aus 15 Gew.-% Nickel auf einem Al₂O₃-haltigen Träger. Der Kupferkatalysator für die Methanolsynthese enthält 60 Gew.-% Kupfer, 30 Gew.-% Zinkoxid und 10 Gew.-% Aluminiumoxid und arbeitet bei einem Druck von 80 bar. Die Trenneinrichtung (15) ist eine Druckwechseladsorptionsanlage, für die eine H₂-Ausbeute von 85 % angenommen wird.

### Beispiel 1:

Man arbeitet mit einer Anlage, die der Zeichnung entspricht; das Erdgas der Leitung (4) ist auf 500°C vorgewärmt, ebenso der technisch reine Sauerstoff der Leitung (5) und der Wasserdampf der Leitung (6). Es werden dem Brenner (3) pro Stunde 1472 kmol Erdgas, 1946 kmol Wasserdampf und 850 kmol O₂ zugeführt. Das Erdgas enthält außer Methan 1,5 Mol % C₂H₆ und 0,4 Mol % C₃H₈ + C₄H₁₀. Weitere Einzelheiten ergeben sich aus der nachfolgenden Tabelle I, wobei in verschiedenen Leitungen die Komponenten des jeweiligen Gemisches in Mol % und kmol/h angegeben sind:

**Tabelle I**

| Leitung | 8 | | 14 | 16 | 33 | | 34 |
|---|---|---|---|---|---|---|---|
| | Mol% | kmol/h | Mol% | Mol% | Mol% | kmol/h | kmol/h |
| CO₂ | 6,1 | 383 | -- | 8 | 12 | 60 | 60 |
| CO | 17 | 1070 | -- | 22,4 | 4,9 | 24 | 24 |
| H₂ | 47,3 | 2982 | 100 | 69 | 73,4 | 365 | 56 |
| CH₄ | 0,8 | 50 | -- | 1 | 8,9 | 44 | 44 |
| H₂O | 28,8 | 1821 | -- | -- | -- | -- | -- |
| CH₃OH | -- | -- | -- | -- | 0,3 | 2 | 2 |
| Gesamtmenge (kmol/h) | 6310 | | 310 | 4800 | 498 | | 188 |
| Temperatur (°C) | 1000 | | 35 | 35 | 35 | | 35 |
| Druck (bar) | 35 | | 83 | 82 | 79 | | 5 |

Die Stöchiometriezahl S beträgt im rohen Synthesegas der Leitung (8) 1,79 und im Gas der Leitung (16) ist S = 2,0.

### Beispiel 2:

Der Druck im Reaktor (1) beträgt nunmehr 50 bar, so daß der Verdichter (12) entfallen kann. Es wird das gleiche Erdgas wie im Beispiel 1 verwendet, die Temperatur in den Leitungen (4), (5) und (6) beträgt jeweils 600°C. Dem Brenner (3) führt man pro Stunde 1498 kmol Erdgas, 1982 kmol Wasserdampf und 800 kmol technisch reinen Sauerstoff zu. Weitere Daten ergeben sich aus Tabelle II:

**Tabelle II**

| Leitung | 8 | | 14 | 16 | 33 | | 34 |
|---|---|---|---|---|---|---|---|
| | Mol% | kmol/h | Mol% | Mol% | Mol% | kmol/h | kmol/h |
| CO₂ | 5,9 | 372 | -- | 7,8 | 10,7 | 50 | 50 |
| CO | 16,7 | 1057 | -- | 22,2 | 6,3 | 30 | 30 |
| H₂ | 47,3 | 2993 | 100 | 67,8 | 61,6 | 289 | 54 |
| CH₄ | 1,7 | 103 | -- | 2,2 | 20,8 | 95 | 95 |
| H₂O | 28,4 | 1794 | -- | -- | 0,1 | -- | -- |
| CH₃OH | -- | -- | -- | -- | 0,5 | 2 | 2 |
| Gesamtmenge (kmol/h) | 6233 | | 235 | 4764 | 470 | | 234 |
| Temperatur (°C) | 1000 | | 35 | 35 | 35 | | 35 |
| Druck (bar) | 50 | | 53 | 52 | 49 | | 5 |
| S | 1,83 | | | 2 | | | |

### 1. Vergleichsbeispiel (gerechnet):

Für die Erzeugung von 1000 Tagestonnen Methanol verwendet man das Erdgas des Beispiels 1, vorgewärmt auf 500°C, und leitet davon 360 kmol/h durch einen kommerziellen Röhrenofen zur Dampfreformierung an einem üblichen Nickelkatalysator. Man erzeugt so ein H₂-haltiges Primärgas mit etwa 40 Vol.% H₂, welchem man 1030 kmol/h Erdgas zumischt. Das Gemisch gibt man einem autotherm betriebenen Reaktor auf, wie er im erfindungsgemäßen Verfahren der Zeichnung allein nur verwendet wird. Unter Zugabe von O₂ und Wasserdampf entsteht ein rohes Synthesegas in einer Gesamtmenge von 5900 kmol/h, dessen Temperatur 1000°C und dessen Druck 35 bar beträgt, wobei S = 1,95 ist. Durch die Leitung (14) mischt man diesem Gas 66 kmol/h Wasserstoff zu und erhöht so S auf 2,0. Die Purgegasmenge (Leitung 33) beträgt nur 147 kmol/h, die einer Druckwechseladsorptionsanlage aufgegeben wird. Durch die Leitung (34) werden 81 kmol/h Abgas abgeführt, welches sich zusammensetzt aus (in kmol/h) 8,2 CO₂, 4,1 CO,21,1 H₂, 0,5 Methanol, 2,4 N₂ und 44,7 CH₄. Es ist festzustellen, daß insbesondere die Menge und die Zusammensetzung des Purgegases erheblich anders als in den Beispielen 1 und 2 sind.

### 2. Vergleichsbeispiel (gerechnet):

Man verwendet das Erdgas des Beispiels 1 und die Verfahrensführung der Zeichnung. Aus dem rohen Synthesegas der Leitung (11) werden 20% des CO₂ entfernt, so daß die Stöchiometriezahl auf 1,93 ansteigt. In verschiedenen Leitungen hat man folgende Gasmengen und Gaszusammensetzungen:

| Leitung | 11 | | 14 | 16 | 33 | | 34 |
|---|---|---|---|---|---|---|---|
| | Mol% | kmol/h | Mol% | Mol% | Mol% | kmol/h | kmol/h |
| CO₂ | 7,1 | 307 | -- | 6,9 | 6,1 | 9 | 9 |
| CO | 24,2 | 1050 | -- | 23,7 | 3 | 5 | 5 |
| H₂ | 67,5 | 2924 | 100 | 68,2 | 65,8 | 98 | 3 |
| CH₄ | 1,1 | 49 | -- | 1,1 | 23,1 | 35 | 35 |
| CH₃OH+N₂ | -- | -- | -- | -- | 2 | 3 | 3 |
| Gesamtmenge (kmol/h) | 4330 | | 95 | 4425 | 15 | | 55 |
| Temperatur (°C) | 35 | | 35 | 35 | 35 | | 35 |
| Druck (bar) | 34 | | 83 | 82 | 79 | | 5 |
| S | 1,93 | | | 2 | | | |

Auch hier ist die Zusammensetzung und die Menge des Purgegases in der Leitung (33) erheblich anders als in den Beispielen 1 und 2.

## Patentansprüche

1. Verfahren zum Erzeugen von Methanol aus Erdgas, wobei man das Erdgas mit Wasserdampf und molekularem Sauerstoff katalytisch umsetzt und ein rohes Synthesegas aus der katalytischen Umsetzung abzieht, welches H₂, CO und CO₂ enthält, wobei man dem rohen Synthesegas ein H₂-reiches Gas und Kreislaufgas zumischt und ein Synthesegasgemisch erzeugt, welches man durch mindestens einen Methanolsynthesereaktor (22) leitet, der einen Kupferkatalysator enthält und bei einem Druck im Bereich von 10 bis 150 bar und Temperaturen im Bereich von 180 bis 350°C betrieben wird, wobei man aus dem Methanolsynthesereaktor (22) ein Methanoldampf enthaltendes Gasgemisch abzieht, welches man soweit kühlt, daß Methanol auskondensiert, wobei ein gekühltes Restgas getrennt vom Methanol abgezogen und in das Kreislaufgas und in einen zweiten Teilstrom (Purgegas) geteilt wird, wobei man aus dem Purgegas, welches einen H₂-Gehalt von mindestens 30 Vol.% (trocken gerechnet) aufweist, in einer Trenneinrichtung (15) ein H₂-reiches Gas abtrennt und es dem rohen Synthesegas zumischt, **dadurch gekennzeichnet, daß**
(a) man die katalytische Umsetzung des Erdgases mit dem Wasserdampf und dem Sauerstoff nur im autotherm betriebenen Reaktor (1) durchführt, der mindestens einen, mit dem Erdgas und dem Sauerstoff gespeisten Brenner (3) und unter dem Brenner (3) eine Schüttung (2) aus körnigem Katalysator aufweist;
(b) man das rohe Synthesegas mit einer Temperatur im Bereich von 800 bis 1200°C aus dem autotherm betriebenen Reaktor (1) abzieht, kühlt, Wasserdampf auskondensiert und gebildetes Kondensat abtrennt;
(c) das rohe, gekühlte Synthesegas vor der Zumischung des H₂reichen Gases eine Stöchiometriezahl S von 1,3 bis 1,9 hat, wobei sich S aus den molaren Konzentrationen von H₂, CO und CO₂ gemäß
S = (H₂ - CO₂) : (CO + CO₂) errechnet;
(d)
das Purgegas zu 30 bis 80 Vol.% aus H₂
zu 7 bis 35 Vol.% aus CO₂ und
zu 3 bis 25 Vol.% aus CO besteht,
(e) die stündliche Menge an Purgegas 8 bis 25 % (trocken gerechnet) der stündlichen Menge an gekühltem, rohem Synthesegas beträgt;
(f) die im Purgegas zur Trenneinrichtung (15) geführte stündliche Menge an Kohlendioxid 10 bis 50 % der stündlichen Menge an CO₂ im gekühlten, rohen Synthesegas beträgt;
(g) die im Purgegas zur Trenneinrichtung (15) geführte stündliche Menge an Kohlenmonoxid 2 bis 10 % der stündlichen Menge an CO im gekühlten, rohen Synthesegas beträgt und
(h) das aus der Trenneinrichtung (15) abgezogene, dem rohen Synthesegas zugemischte H₂-reiche Gas einen H₂-Gehalt von mindestens 80 Vol.% hat, wobei durch die H₂-Zumischung im Synthesegas eine Stöchiometriezahl von 1,95 bis 2,2 erreicht wird.

2. Verfahren nach Anspruch 1, **dadurch gekennzeichnet, daß** das aus der Trenneinrichtung (15) abgezogene und dem rohen Synthesegas zugemischte H₂-reiche Gas einen H₂-Gehalt von mindestens 90 Vol.% hat.

3. Verfahren nach Anspruch 1 oder 2, **dadurch gekennzeichnet, daß** man den autotherm betriebenen Reaktor (1) bei einem Druck oberhalb von mindestens 40 bar betreibt und das rohe Synthesegas ohne Verwendung eines Gasverdichters zur Methanolsynthese leitet.

## Claims

1. A process of producing methanol from natural gas, where the natural gas is catalytically converted with steam and molecular oxygen, and a raw synthesis gas is withdrawn from the catalytic conversion, which gas contains H₂, CO and CO₂, a gas rich in H₂ and recycle gas are admixed to the raw synthesis gas and a synthesis gas mixture is produced, which is passed through at least one methanol synthesis reactor (22) which contains a copper catalyst and is operated at a pressure in the range from 10 to 150 bar and at temperatures in the range from 180 to 350°C, a gas mixture containing methanol vapour being withdrawn from the methanol synthesis reactor (22), which mixture is cooled to such an extent that methanol condenses out, a cooled residual gas being separately withdrawn from the methanol and divided into the recycle gas and a second partial stream (purge gas), a gas rich in H₂ being separated from the purge gas, which has an H₂ content of at least 30% by volume (calculated dry), in a separating means (15) and admixed to the raw synthesis gas, **characterised in that**
a) the catalytic conversion of the natural gas with the steam and the oxygen is performed only in the autothermally operated reactor (1), which comprises at least one burner (3) fed with the natural gas and the oxygen, and below the burner (3) a bed (2) of granular catalyst;
b) the raw synthesis gas is withdrawn from the autothermally operated reactor (1) at a temperature in the range from 800 to 1200°C, is cooled, steam is condensed out, and resulting condensate is separated;
c) the raw, cooled synthesis gas has a stoichiometric number S of 1.3 to 1.9 prior to admixing the gas rich in H₂, where S is calculated from the molar concentrations of H₂, CO and CO₂ in accordance with
S = (H₂ - CO₂) : (CO + CO₂);
d)
the purge gas consists of 30 to 80% by volume H₂,
7 to 35% by volume CO₂, and
3 to 25% by volume CO,
e) the hourly quantity of purge gas is 8 to 25% (calculated dry) of the hourly quantity of cooled, raw synthesis gas;
f) the hourly quantity of carbon dioxide supplied to the separating means (15) in the purge gas is 10 to 50% of the hourly quantity of CO₂ in the cooled, raw synthesis gas;
g) the hourly quantity of carbon monoxide supplied to the separating means (15) in the purge gas is 2 to 10% of the hourly quantity of CO in the cooled, raw synthesis gas; and
h) the gas rich in H₂, which was withdrawn from the separating means (15) and admixed to the raw synthesis gas, has an H₂ content of at least 80% by volume, and by admixing H₂ a stoichiometric number of 1.95 to 2.2 is achieved for the synthesis gas.

2. A process according to claim 1, **characterised in that** the gas rich in H₂ withdrawn from the separating means (15) and admixed to the raw synthesis gas has an H₂ content of at least 90% by volume.

3. A process according to claim 1 or 2, **characterised in that** the autothermally operated reactor (1) is operated at a pressure above at least 40 bar, and the raw synthesis gas is supplied to the methanol synthesis without use of a gas compressor.

## Revendications

1. Procédé de production de méthanol à partir de gaz naturel, dans lequel on fait réagir catalytiquement le gaz naturel sur de la vapeur d'eau et de l'oxygène moléculaire et l'on soutire de la réaction catalytique un gaz de synthèse brut qui contient H₂, CO et CO₂, on ajoute au gaz de synthèse brut un gaz riche en H₂ et un gaz de circulation et on produit un mélange de gaz de synthèse que l'on envoie dans au moins un réacteur (22) de synthèse du méthanol qui contient un catalyseur au cuivre et qui fonctionne sous une pression de l'ordre de 10 à 150 bars et à des températures de l'ordre de 180 à 350°C, on soutire du réacteur (22) de synthèse du méthanol un mélange gazeux contenant de la vapeur de méthanol que l'on refroidit jusqu'à ce que le méthanol se condense, on soutire un gaz résiduel refroidi séparément du méthanol et on l'envoie dans le gaz de circuit et dans un deuxième courant partiel (gaz de purge), on sépare du gaz de purge, qui a une teneur en H₂ d'au moins 30 % en volume (calculé en matière sèche), dans un dispositif (15) de séparation un gaz riche en H₂ et on le mélange au gaz de synthèse brut, **caractérisé en ce que**
(a) on effectue la réaction catalytique du gaz naturel sur la vapeur d'eau et l'oxygène seulement dans un réacteur (1) fonctionnant de manière autothermique qui comporte au moins un brûleur (3) alimenté en gaz naturel et en oxygène et sous le brûleur (3) un tas (2) de catalyseur en grains ;
(b) on soutire le gaz de synthèse brut ayant une température de l'ordre de 800 à 1200°C du réacteur (1) fonctionnant de manière autothermique, on le refroidit, on en condense la vapeur d'eau et on sépare le produit condensé formé ;
(c) le gaz de synthèse brut refroidi a, avant l'addition du gaz riche en H₂, un indice stoechiométrique S de 1,3 à 1,9, S étant calculé à partir des concentrations molaires de H₂, CO et CO₂ suivant
S = (H₂ - CO₂) : (CO + CO₂) ;
(d)
le gaz de purge est constitué pour 30 à 80 % en volume d'H₂
pour 7 à 35% en volume de CO₂ et
pour 3 à 25 % en volume de CO,
(e) la quantité horaire de gaz de purge représente de 8 à 25 % (exprimé en matière sèche) de la quantité horaire du gaz de synthèse brut refroidi ;
(f) la quantité horaire de dioxyde de carbone envoyée dans le gaz de purge au dispositif (15) de séparation représente de 10 à 50 % de la quantité horaire de CO₂ dans le gaz de synthèse brut refroidi ;
(g) la quantité horaire de monoxyde de carbone envoyée dans le gaz de purge au dispositif (15) de séparation représente de 2 à 10 % de la quantité horaire de CO dans le gaz de synthèse brut refroidi et
(h) le gaz riche en H₂ soutiré du dispositif (15) de séparation et ajouté au gaz de synthèse brut a une teneur en H₂ d'au moins 80 % en volume, un indice de stoechiométrie de 1,95 à 2,2 étant obtenu par l'addition d'H₂ au gaz de synthèse.

2. Procédé suivant la revendication 1, **caractérisé en ce que** le gaz riche en H₂ soutiré du dispositif (15) de séparation et ajouté au gaz de synthèse brut a une teneur en H₂ d'au moins 90 % en volume.

3. Procédé suivant la revendication 1 ou 2, **caractérisé en ce que** l'on fait fonctionner le réacteur (1) qui fonctionne de manière autothermique à une pression supérieure à au moins 40 bars et on envoie le gaz de synthèse brut à la synthèse du méthanol sans utilisation d'un compresseur de gaz.
